## Europäisches Patentamt

## European Patent Office

## Office européen des brevets

(19)

(11) Veröffentlichungsnummer: **0 083 433**
**B1**

(12) **EUROPÄISCHE PATENTSCHRIFT**

(45) Veröffentlichungstag der Patentschrift:
**23.10.85**

(51) Int. Cl.4: **C 07 C 7/12,** C 07 C 11/04,
B 01 D 53/04

(21) Anmeldenummer: **82111680.3**

(22) Anmeldetag: **16.12.82**

(54) **Adsorptionsverfahren zur Trennung von Kohlenwasserstoffen.**

(30) Priorität: **18.12.81 DE 3150137**

(43) Veröffentlichungstag der Anmeldung:
**13.07.83 Patentblatt 83/28**

(45) Bekanntmachung des Hinweises auf die Patenterteilung:
**23.10.85 Patentblatt 85/43**

(84) Benannte Vertragsstaaten:
**AT BE DE FR GB IT NL**

(56) Entgegenhaltungen:
**DE - A - 2 916 585**
**DE - B - 2 461 759**
**GB - A - 2 058 826**

(73) Patentinhaber: **Linde Aktiengesellschaft,
Abraham-Lincoln-Strasse 21, D-6200 Wiesbaden (DE)**
Patentinhaber: **CHEMISCHE WERKE HÜLS AG,
Postfach 1320, D-4370 Marl 1 (DE)**

(72) Erfinder: **Benkmann, Christian, Dipl.-Ing.,
Meisenstrasse 39, D-8032 Gräfelfing (DE)**

(74) Vertreter: **Schaefer, Gerhard, Dr., Linde
Aktiengesellschaft Zentrale Patentabteilung,
D-8023 Höllriegelskreuth (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft ein Verfahren zur Gewinnung eines Kohlenwasserstoffs aus einem Sauerstoff enthaltenden Gasstrom nach dem Druckwechseladsorptionsverfahren in einer Anzahl von im zyklischen Wechsel betriebenen Adsorbern, in denen während einer Adsorptionsphase bei erhöhtem Druck eine selektive Adsorption des zu gewinnenden Kohlenwasserstoffs erfolgt und während einer Gegenstromentspannung vom Eintrittsende des Adsorbers ein an dem zu gewinnenden Kohlenwasserstoff angereicherter Desorbatstrom abgezogen wird und der Adsorber nach der Desorption eine Druckaufbauphase durchläuft.

Aus der DE-OS 30 35 255 ist schon ein Verfahren zur Gewinnung oder Rückgewinnung von Kohlenwasserstoffen aus einem Kohlenwasserstoffe und ein Trägergas enthaltenden Gasstrom bekannt, bei dem insbesondere Methan in einer Druckwechseladsorptionsanlage während einer Adsorptionsphase aus einem Methan-Luft-Gemisch abgetrennt wird. Während aus dem Austrittsende der Adsorber dieser Anlage weitgehend methanfreie Luft austritt, wird angereichertes Methan während einer Regenerierphase der Adsorber als Desorbatstrom gewonnen.

Dieses Verfahren ist auf die vollständige Abtrennung aller Kohlenwasserstoffe ausgerichtet, insbesondere auf die vollständige Abtrennung von Methan aus einem Methan-Luft-Gemisch. Das dafür vorgeschlagene Adsorptionsverfahren enthält als wesentlichen Verfahrensschritt eine auf eine Adsorptionsphase folgende Nachbeladung mit gut adsorbierbaren Komponenten, insbesondere mit dem als Produkt zu gewinnenden Methan. Eine solche Nachbeladung, die bei Adsorptionsdruck, also dem höchsten Verfahrensdruck, vorgenommen wird, ist jedoch mit Nachteilen behaftet, weil hierzu eine Rückverdichtung des Methans auf den Adsorptionsdruck erforderlich ist.

Die Kohlenwasserstoffkonzentration in den in der DE-OS 30 35 255 betrachteten Anwendungsfällen ist relativ niedrig; es werden Methankonzentrationen zwischen 1 und 40 Vol.-% im Rohgas angegeben. Da die weitere Komponente Luft sein soll, ergeben sich im Rohgas Sauerstoffkonzentrationen von etwa 12 bis 20 Vol-%. Bei einem so hohen Sauerstoffgehalt besteht jedoch eine sehr große Explosionsgefahr des Gemisches, so daß ein sicherer Betrieb einer solchen Anlage als unmöglich erscheint.

Auch bei der Verarbeitung von sauerstoffarmen Rohgasen besteht die Gefahr, daß in der Adsorptionsanlage Sauerstoffkonzentrationen auftreten, die das Kohlenwasserstoff-Sauerstoff-Gemisch in den explosiblen Bereich gelangen lassen. Der vorliegenden Erfindung liegt deshalb die Aufgabe zugrunde, ein Verfahren der eingangs genannten Art so auszugestalten, daß die Gewinnung einzelner Kohlenwasserstoffe aus einem mit geringen Mengen an Sauerstoff verunreinigtem Kohlenwasserstoffgemisch auf sichere Weise möglich ist.

Diese Aufgabe wird dadurch gelöst, daß zur Gewinnung eines Kohlenwasserstoffs aus einem geringe Mengen an Sauerstoff enthaltenden Kohlenwasserstoffgemisch während der Adsorptionsphase und während mindestens einer an die Adsorptionsphase anschließenden Gleichstromentspannungsphase am Austrittsende eines Adsorbers ein kohlenwasserstoffhaltiger Gasstrom abgezogen wird, der an dem zu gewinnenden Kohlenwasserstoff verarmt ist, und daß zum Druckaufbau eines Adsorbers nach der Desorption ein Gas verwendet wird, dessen Sauerstoffgehalt geringer ist als der Sauerstoffgehalt der während einer Adsorptionsphase oder einer Gleichstromentspannungsphase aus einem Adsorber abströmenden Gase.

Beim erfindungsgemäßen Verfahren sollen kohlenwasserstoffreiche Gasgemische verarbeitet werden, deren Sauerstoffgehalt so niedrig liegt, daß auch keine Explosionsgefahr besteht. Die hierfür erforderliche Begrenzung der Sauerstoffkonzentration hängt im Einzelfall von der jeweiligen Gaszusammensetzung sowie von den Verfahrensbedingungen, unter denen das Gas anfällt und verarbeitet wird, ab. Beispielsweise liegt die Explosionsgrenze für Gase mit überwiegendem Anteil an Methan und Äthylen bei einem Druck vo 10 bar bei einer Sauerstoffkonzentration von etwa 10%, bei einem Druck von 20 bar dagegen bei einer Sauerstoffkonzentration von etwa 16%. Allgemein sollte für einen sicheren Betrieb der Sauerstoffgehalt unter 15%, vorzugsweise unter 12%, insbesondere unter 10% gehalten werden.

Bei der Durchführung eines Druckwechseladsorptionsverfahrens werden in den Adsorbern einzelne Komponenten zurückgehalten, so daß sich sowohl eine lokale als auch eine zeitliche Änderung der Gaszusammensetzung ergibt. Dabei ist für eine sichere Durchführung des Verfahrens darauf zu achten, daß zu keiner Zeit und an keiner Stelle ein Überschreiten der höchst zulässigen Sauerstoffkonzentration erfolgt.

Für die Durchführung der Druckwechseladsorption haben sich Verfahren als besonders günstig erwiesen, in denen für das Aufdrücken eines regenerierten Adsorbers zumindest teilweise das Gas verwendet wird, das während einer Entspannungsphase im Anschluß an die Adsorptionsphase aus dem Adsorber abgezogen wird. Dies ist üblicherweise ein im Gleichstrom mit der Adsorptionsrichtung aus dem Adsorber abgezogenes Entspannungsgas, dessen Zusammensetzung weitgehend der Zusammensetzung des während der Adsorption abgezogenen, nicht adsorbierten Gasstroms entspricht. Eine solche Verfahrensweise nutzt in energetisch günstiger Weise das Druckpotential der Entspannungsgase aus und führt darüber hinaus zu einer erhöhten Ausbeute an der zu gewinnenden Komponente, die üblicherweise der nicht adsorbierte Verfahrensstrom ist. Verfahren dieser Art sind beispielsweise beschrieben in der DE-PS 17 69 936, der DE-OS 28 40 357 oder der DE-OS 29 16 585.

Es hat sich nun gezeigt, daß eine Übertragung dieser an sich bewährten Verfahren auf die Abtrennung von einzelnen Kohlenwasserstoffen aus Kohlenwasserstoffgemischen, die mindestens noch Sauerstoff enthalten, in vielen Fällen nicht möglich ist, weil dabei unzulässig hohe Sauerstoffkonzentrationen auftreten.

Beim erfindungsgemäßen Verfahren wird mindestens ein Kohlenwasserstoff selektiv adsorbiert, während andere Kohlenwasserstoffe, Sauerstoff und gegebenenfalls im Gas enthaltene weitere Komponenten den Adsorber durchströmen und in angereicherter Form aus dem Adsorber austreten. Während einer an die Adsorptionsphase anschließenden Gleichstromentspannung tritt dann ebenfalls ein Gas mit einer vergleichbaren Zusammensetzung aus dem Adsorber aus. Auch in diesen angereicherten Gasen, in denen der gesamte im Rohgas enthaltene Sauerstoff anfällt, muß noch ein unterhalb der Explosionsgrenze liegender Sauerstoffgehalt eingehalten werden. Erfindungsgemäß wesentlich ist jedoch, daß dieses Gas dennoch nicht zum Druckaufbau eines regenerierten Adsorbers verwendet werden darf. Es hat sich nämlich gezeigt, daß beim Einleiten dieses Gases in einen aufzudrückenden Adsorber eine lokale Sauerstoffkonzentration aufgebaut wird, die leicht zu einer Überschreitung der höchst zulässigen Sauerstoffkonzentration führt. Dies liegt vor allem daran, daß sich im aufzudrückenden Adsorber keine einheitliche Sauerstoffverteilung einstellt, sondern daß sich in Richtung auf das Austrittsende des Adsorbers hin eine zunehmende Sauerstoffkonzentration ergibt.

Beim erfindungsgemäßen Verfahren wird deshalb der Druckaufbau eines regenerierten Adsorbers mit einem sauerstoffarmen Gas vorgenommen. Als ein solcher Gasstrom eignet sich in vielen Fällen der zu zerlegende Gasstrom, dessen Sauerstoffgehalt geringer ist als der des aus dem Adsorberende austretenden Gases. Gegebenenfalls kann das Aufdrücken jedoch auch mit anderen geeigneten und verfügbaren Gasströmen, beispielsweise Methan, vorgenommen werden. Der Einsatz von sauerstofffreien Fremdgasen wie Methan ist insbesondere dann günstig, wenn im Gasstrom schon eine relativ hohe Sauerstoffkonzentration vorliegt.

Auch beim Aufdrücken der Adsorber mit einem sauerstoffarmen Gas, wie es das zu zerlegende Gasgemisch darstellt, ergibt sich im Adsorber eine ungleichmäßige Verteilung der Sauerstoffkonzentration. Aus dem zum Aufrücken verwendeten Rohgas werden die zu gewinnenden Kohlenwasserstoffe unter Ausbildung einer sich auf das Austrittsende hin bewegenden Adsorptionsfront selektiv abgetrennt, während die nicht adsorbierten Bestandteile sofort bis ans verschlossene Austrittsende des Adsorbers vordringen können. Damit ergibt sich aber durch die Zurückhaltung einzelner Komponenten ein Konzentrationsanstieg des Sauerstoffs zum Austrittsende des Adsorbers hin. Der Anstieg der Sauerstoffkonzentration hängt dabei unter anderem auch von der Selektivität des Adsorptionsmittels für den zu gewinnenden Kohlenwasserstoff ab. So ist beispielsweise bei der Zerlegung eines überwiegend Methan und Äthylen enthaltenden Gasstroms und bei Verwendung von Aktivkohle als Adsorptiosmittel neben einer gewünschten Adsorption von Äthylen auch die Coadsorption von Methan relativ groß, wodurch sich eine stärkere Sauerstoffkonzentration am Austrittsende des Adsorbers einstellt als beispielsweise bei der Verwendung von Silikagel als Adsorptionsmittel.

In Weiterbildung des erfindungsgemäßen Verfahrens wird zur Vermeidung unerwünscht oder gefährlich hoher Sauerstoffkonzentrationen am Austrittsende des Adsorbers während einer Aufdrückphase vorgeschlagen, daß schon vor Beendigung der Druckaufbauphase über das Austrittsende des Adsorbers Gas abgezogen wird. Durch diese Maßnahme werden die gefährlichen Sauerstoffspitzenkonzentrationen schon während ihrer Entstehung abgebaut. Bei der Gewinnung von Äthylen aus im wesentlichen Methan und Äthylen enthaltenden Gasströmen empfiehlt sich diese Verfahrensweise insbesondere dann, wenn mit Aktivkohle als Adsorptionsmittel gearbeitet wird. Bei der Verwendung von Silikagel als Adsorptionsmittel ist diese Verfahrensvariante aus Sicherheitsgründen nicht nötig.

Im Anschluß an eine Adsorptionsphase wird beim erfindungsgemäßen Verfahren eine Gleichstromentspannung durchgeführt, während der ein Gleichstromentspannungsgas abgezogen wird, das im wesentlichen frei von der zu adsorbierenden Komponente ist und das aus den Hohlräumen in der Adsorptionsmittelschüttung während der Gleichstromentspannung entweicht. Die Adsorptionsphase wird zu einem Zeitpunkt abgebrochen, an dem die Adsorptionsfront noch nicht das Austrittsende des Adsorbers erreicht hat, so daß während der Gleichstromentspannungsphase die in dem Gasgemisch, das das Porenvolumen ausfüllt, noch enthaltenen zu gewinnenden Komponenten unter weiterem Fortschreiten der Adsorptionsfront auf das Austrittsende des Adsorbers zu abgetrennt werden. Das während der Gleichstromentspannungsphase anfallende, einen höheren Sauerstoffgehalt als das Rohgas aufweisende Gasgemisch darf zwar nicht zum Aufdrücken eines regenerierten Adsorbers verwendet werden, kann aber wegen seiner geringen Konzentration an der zu gewinnenden Komponente zur Spülung eines Adsorbers vor der Aufdrückphase verwendet werden. Eine solche Spülung kann sich an die Gegenstromentspannung des Adsorbers, während der die eigentliche Produktfraktion abgezogen wird, anschließen, um gegebenenfalls noch eine Restbeladung des Adsorbers abzubauen. Eine Spülung des Adsorbers ist jedoch nicht in allen Fällen erforderlich.

Der während der Gegenstromentspannung anfallende Desorbatstrom enthält den zu gewinnenden Kohlenwasserstoff in einer Konzentration, die sowohl von der Konzentration im Rohgas als auch von der Selektivität des Adsorptionsmittels abhängt. Falls die Konzentration der gewünschten Produktkomponente in diesem Gasstrom noch unzureichend ist, kann dieser Desorbatstrom in einer weiteren Trennvorrichtung in einen hoch angereicherten Strom des zu gewinnenden Kohlenwasserstoffs und in einen Restgasstrom zerlegt werden. Für eine solche nachfolgende Zerlegung eignet sich beispielsweise eine weitere Druckwechsel-Adsorptionsanlage.

In manchen Fällen ist es möglich, daß der zu zerlegende Rohgasstrom Komponenten enthält, die besser als der Kohlenwasserstoff, dessen Gewinnung beabsichtigt ist, adsorbierbar sind. In einem solchen Fall werden in weiterer Ausgestaltung des erfindungsgemäßen Verfahrens die besser adsorbierbaren Komponenten in vorgeschalteten Adsorbern, gegebenenfalls mit einem anderen Adsorptionsmittel, abgetrennt. Derartige vorgeschaltete Adsorber können sowohl in einer gesonderten Adsorberstation angeordnet sein als auch als Vorbetten zu den eigentlichen Adsorberbetten ausgebildet und mit den Hauptadsorbern in einem gemeinsamen Gehäuse angeordnet sein. Für die Regenerierung der vorgeschalteten Adsorber eignet sich als Spülgas insbesondere der Gasstrom, der aus dem Austrittsende der Hauptadsorber austritt. Diese Verfahrensweise ist insbesondere dann günstig, wenn das nicht adsorbierte Gas als geringwertiges Restgas, das beispielsweise nur zu Heizzwecken verwendet werden kann, anfällt.

Für das erfindungsgemäße Verfahren eignen sich insbesondere Adsorptionsanlagen mit mindestens drei Adsorbern, vorzugsweise mit mindestens vier Adsorbern.

Weitere Einzelheiten der Erfindung werden nachfolgend anhand eines Ausführungsbeispiels, das in den Figuren schematisch dargestellt ist, beschrieben.

Es zeigt

Figur 1 eine Anlage zur Durchführung des erfindungsgemäßen Verfahrens und

Figur 2 ein Zeitablaufschema für den Betrieb der Anlage gemäß Figur 1.

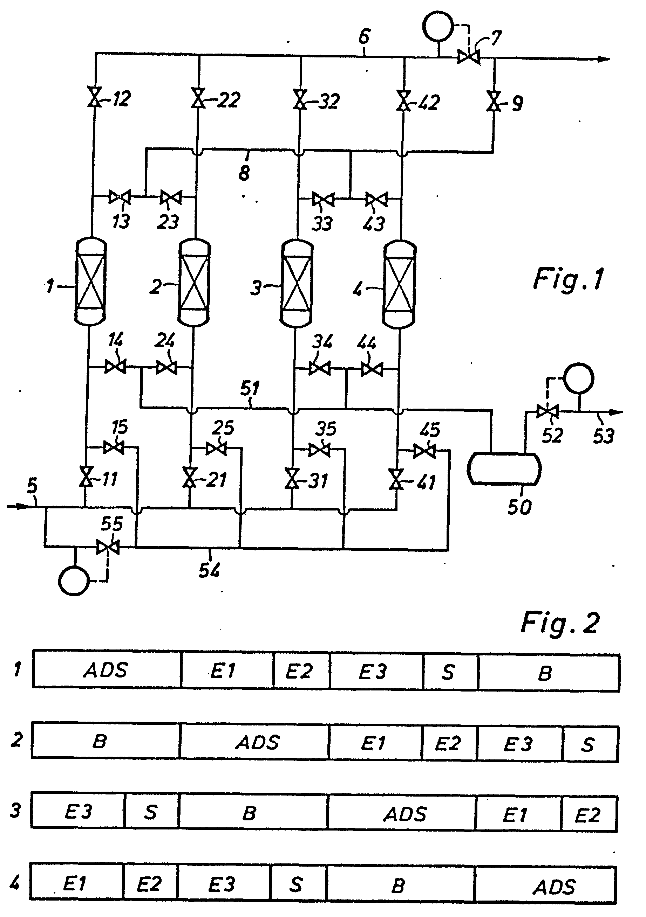

Die in Figur 1 dargestellte Druckwechsel-Adsorptionsanlage besteht aus vier Adsorbern 1, 2, 3 und 4. Die Adsorber sind über Ventile 11, 21, 31 und 41 eintrittsseitig mit einer Rohgasleitung 5 verbunden und austrittsseitig über Ventile 12, 22, 32 und 42 mit einer Restgasleitung 6, über die die nicht adsorbierten Komponenten des Rohgases abgezogen werden. Vor der Abgabe des Restgases wird der Druck im regelbaren Entspannungsventil 7 reduziert. Weiterhin sind die Austrittsenden der Adsorber über die Ventile 13, 23, 33 bzw. 43 mit einer Leitung 8 verbunden, die über das Ventil 9 mit der Restgasleitung in Verbindung steht. Über diese Leitung werden während einer ersten Entspannungsphase anfallende, an nicht adsorbierten Komponenten reiche Entspannungsgase ins Restgas abgeführt. Auf der Eintrittsseite der Adsorber sind Ventile 14, 24, 34 bzw. 44 vorgesehen, die das Eintrittsende der Adsorber mit einer zum Pufferbehälter 50 führenden Leitung 51 verbinden. Der Puffer 50 ist über ein Regelventil 52 mit der Produktleitung 53 verbunden. Über die Leitung 51 wird dem Puffer das während einer Gegenstromentspannung anfallende Produktgas sowie während einer Spülphase anfallendes Spülgas und Desorbat zugeleitet.

Schließlich sind auf der Eintrittsseite der Adsorber noch Ventile 15, 25, 35 und 45 angeordnet, die über Leitung 54 und dem Regelventil 55 mit der Rohgasleitung 5 verbunden sind. Über diese Leitung erfolgt der Druckaufbau der gespülten Adsorber mittels Rohgas.

In Figur 2 ist das Zeitablaufschema für den Betrieb der Anlage dargestellt. Die vier Adsorber durchlaufen identische Zyklen, die zeitlich so gegeneinander versetzt sind, daß jeweils ein Adsorber in der Adsorptionsphase ist, so daß ein kontinuierlicher Betrieb gewährleistet ist. An die bei konstantem Druck durchgeführte Adsorptionsphase ADS schließt sich eine erste Entspannung E1 im Gleichstrom an. Das dabei anfallende Entspannungsgas wird ins Restgas abgeführt. In einer zweiten Gleichstromentspannungsphase E2 wird weiteres Gas über das Austrittsende des Adsorbers abgezogen. Dieses Gas gelangt über die geöffneten Ventile 13 und 43 ans Austrittsende des Adsorbers 4 und durchströmt den in einer Spülphase S befindlichen Adsorber 4, bevor es über Ventil 44 und Leitung 51 in den Pufferbehälter 50 gelangt. Im Anschluß an die zweite Gleichstromentspannung folgt eine Gegenstromentspannungsphase E3, während der ein Desorbatstrom über das geöffnete Ventil 14 und die Leitung 51 zum Pufferbehälter 50 geleitet wird. Nach Beendigung der Gegenstromentspannung E3 wird der Adsorber einer Spülphase S unterworfen. Dazu wird Entspannungsgas aus der Gleichstromentspannungsphase E2 des Adsorbers 2 über die geöffneten Ventile 23 und 13 im Gegenstrom zur Adsorptionsrichtung durch den Adsorber 1 geleitet. Durch das Spülgas erfolgt eine Verdrängung der adsorbierten Produktkomponente, die noch in hoher Konzentration am Eintrittsende des Adsorbers austritt. Die Spülphase wird relativ kurz gehalten, um das zum Pufferbehälter 50 geführte Produktgas nicht unnötig mit Spülgas zu verdünnen. In einer anderen Ausgestaltung des Verfahrens kann statt der Spülung S auch eine Desorption durch Anwendung von unteratmosphärischem Druck durchgeführt werden. Die hierdurch verbesserte Produktqualität wird jedoch mit einem erhöhten Energieaufwand für eine Vakuumpumpe erkauft.

Nach Beendigung der Spülphase S kann der Adsorber wieder auf den Adsorptionsdruck aufgedrückt werden. Dies geschieht während der Druckaufbauphase B, während der über die geöffneten Ventile 55 und 15 Rohgas in den Adsorber 1 geführt wird. Nachdem sich im Adsorber 1 der Rohgasdruck aufgebaut hat, ist für den Adsorber 1 ein vollständiger Zyklus durchlaufen. Die übrigen Adsorber durchlaufen den gleichen Zyklus in zeitlicher Versetzung, wie in Figur 2 dargestellt.

Das erfindungsgemäße Verfahren eignet sich beispielsweise für die Zerlegung von Abgasen aus Anlagen zur Erzeugung von Äthylenoxid, die im wesentlichen wertvolles Äthylen, weniger wertvolles Methan, geringe Mengen Sauerstoff sowie gegebenenfalls noch Inertgase wie Argon, Stickstoff oder Kohlendioxid und weitere leichtere Kohlenwasserstoffen enthalten können. Die Menge des in einem solchen Gasstrom enthaltenen Äthylens hängt dabei von der Verfahrensführung der Äthylenoxiderzeugung ab und liegt typischerweise im Bereich zwischen einigen Zehntel und etwa 35 mol-%, während der Sauerstoffgehalt üblicherweise zwischen etwa 1 und 7 mol-% liegt, insbesondere bei der Erzeugung des Äthylenoxids auf Sauerstoffbasis im Bereich von etwa 5 und 7 mol-%.

**Patentansprüche**

1. Verfahren zur Gewinnung eines Kohlenwasserstoffs aus einem Sauerstoff enthaltenden Gasstrom nach dem Druckwechseladsorptionsverfahren in einer Anzahl von im zyklischen Wechsel betriebenen Adsorbern, in denen während einer Adsorptionsphase bei erhöhtem Druck eine selektive Adsorption des zu gewinnenden Kohlenwasserstoffs erfolgt und während einer Gegenstromentspannung vom Eintrittsende des Adsorbers ein an dem zu gewinnenden Kohlenwasserstoff angereicherter Desorbatstrom abgezogen wird und der Adsorber nach der Desorption eine Druckaufbauphase durchläuft, dadurch gekennzeichnet, daß zur Gewinnung eines Kohlenwasserstoffs aus einem geringe Mengen an Sauerstoff enthaltenden Kohlenwasserstoffgemisch während der Adsorptionsphase und während mindestens einer an die Adsorptionsphase anschließenden Gleichstromentspannungsphase am Austrittsende eines Adsorbers ein kohlenwasserstoffhaltiger Gasstrom abgezogen wird, der an dem zu gewinnenden Kohlenwasserstoff verarmt ist, und daß zum Druckaufbau eines Adsorbers nach der Desorption ein Gas verwendet wird, dessen Sauerstoffgehalt geringer ist als der Sauerstoffgehalt der während einer Adsorptionsphase oder einer Gleichstromentspannungsphase aus einem Adsorber abströmenden Gase.

2. Verfahren nach Anspruch 1, dadurch gekennzeichnet, daß schon vor Beendigung der Druckaufbauphase über das Austrittsende des Adsorbers an dem zu gewinnenden Kohlenwasserstoff verarmtes Gas abgezogen wird.

3. Verfahren nach einem der der Ansprüche 1 oder 2, dadurch gekennzeichnet, daß der Desorbatstrom einer weiteren Druckwechsel-Adsorptionsanlage zugeführt und in einen hochangereicherten Strom des zu gewinnenden Kohlenwasserstoffs und in einen Restgasstrom zerlegt wird.

4. Verfahren nach einem der Ansprüche 1 bis 3, dadurch gekennzeichnet, daß im Gasstrom enthaltene Komponenten, die besser als der zu gewinnende Kohlenwasserstoff adsorbierbar sind, in vorgeschalteten Adsorbern selektiv abgetrennt werden, und daß für die Regenerierung dieser Adsorber das am Austrittsende der Hauptadsorber austretende, an dem zu gewinnenden Kohlenwasserstoff verarmte Gas als Spülgas verwendet wird.

5. Verfahren nach einem der Ansprüche 1 bis 4, dadurch gekennzeichnet, daß Äthylen aus einem überwiegend Methan und Äthylen enthaltenden Gasstrom gewonnen wird.

6. Verfahren nach Anspruch 5, dadurch gekennzeichnet, daß Silikagel oder Aktivkohle als Adsorptionsmittel verwendet wird.

**Claims**

1. A process for recovering a hydrocarbon from a gas stream containing oxygen, by a pressure-alternating adsorption process carried out in a plurality of cyclically alternating adsorbers, in which process, during an adsorption phase at an increased pressure, a selective adsorption of the hydrocarbon to be recovered is effected, in which during a counter-flow expansion, a desorbate stream enriched in the hydrocarbon to be recovered, is withdrawn from the inlet end of the adsorber, and in which the adsorber passes through a pressure build-up phase after the desorption, characterised in that, in order to recover a hydrocarbon from a hydrocarbon mixture which contains small amounts of oxygen, during the adsorption phase and during at least one direct-flow expansion phase which follows the adsorption phase, a hydrocarbon-containing gas stream which is impoverished in respect of the hydrocarbon to be recovered, is removed at the outlet end of an adsorber; and that, in order to build up the pressure of an adsorber after the desorption, a gas is used the oxygen content of which is smaller than the oxygen content of the gases which escape from an adsorber during an adsorption phase or a direct-flow expansion phase.

2. A process as claimed in Claim 1, characterised in that, prior to the termination of the pressure build-up phase, gas which is impoverished in respect of the hydrocarbon to be recovered, is removed at the outlet end of the adsorber.

3. A process as claimed in one of Claims 1 or 2, characterised in that the desorbate stream is supplied to a further pressure-alternating adsorption system and separated into a stream highly-enriched in the hydrocarbon to be recovered, and a residual gas stream.

4. A process as claimed in one of Claims 1 to 3, characterised in that components contained in the gas stream and which can be more easily adsorbed than the hydrocarbon to be recovered, are selectively separated in preceding adsorbers; and that for the regeneration of the adsorbers, the gas which espaces at the output end of the main adsorber and which is impoverished in respect of the hydrocarbon to be recovered, is used as scavenging gas.

5. A process as claimed in one of Claims 1 to 4, characterised in that ethylene is recovered from a gas stream which principally contains methane and ethylene.

6. A process as claimed in Claim 5, characterised in that silica gel or activated carbon is used as adsorbent.

**Revendications**

1. Procédé d'extraction d'un hydrocarbure à partir d'un courant gazeux renfermant de l'oxygène, selon le procédé d'adsorption par alternances de pression dans une pluralité d'adsorbeurs fonctionnant en commutation cyclique, dans lesquels l'hydrocarbure à extraire est adsorbé sélectivement sous une pression élevée, pendant une étape d'adsorption, et dont on retire, pendant une étape de détente avec écoulement à contre-courant, à l'extrémité d'entrée de l'adsorbeur, un courant de désorbat enrichi en hydrocarbure à extraire, l'adsorbeur passant, après la désorption, par une étape de mise ou montée en pression, caractérisé en ce que, pour extraire un hydrocarbure à partir d'un mélange d'hydrocarbures renfermant de faibles quantités d'oxygène, on recueille à l'extrémité de sortie d'un adsorbeur, pendant l'étape d'adsorption et pendant au moins une étape de détente avec écoulement équicourant, qui suit ladite étape d'adsorption, un courant gazeux renfermant des hydrocarbures mais appauvri en l'hydrocarbure à extraire, et en ce que, pour augmenter la pression d'un adsorbeur après la désorption, l'on utilise un gaz dont la teneur en oxygène est inférieure à celle des gaz sortant d'un adsorbeur pendant une étape d'adsorption ou une étape de détente avec écoulement équicourant.

2. Procédé selon la revendication 1, caractérisé en ce que, dès avant l'achèvement de l'étape de mise en pression, on retire à l'extrémité de sortie de l'adsorbeur du gaz pauvre en l'hydrocarbure à extraire.

3. Procédé selon une des revendications 1 ou 2, caractérisé en ce qu'on introduit le courant de désorbat dans une installation supplémentaire d'adsorption par alternances de pression et en ce qu'on sépare ce courant en un courant fortement enrichi en l'hydrocarbure à extraire et un courant de gaz résiduel.

4. Procédé selon une des revendications 1 à 3, caractérisé en ce que des composants présents dans le courant gazeux, qui sont plus facilement adsorbables que l'hydrocarbure à extraire, sont séparés sélectivement dans des adsorbeurs disposés en amont, et en ce qu'on utilise, pour la régénération de ces adsorbeurs, en tant que gaz de rinçage le gaz pauvre en l'hydrocarbure à extraire qui sort de l'extrémité de sortie des adsorbeurs principaux.

5. Procédé selon une des revendications 1 à 4, caractérisé en ce que l'on extrait de l'éthylène à partir d'un courant gazeux renfermant en proportion prépondérante du méthane et de l'éthylène.

6. Procédé selon la revendication 5, caractérisé en ce qu'on utilise du gel de silice ou du charbon actif en tant qu'agent adsorbant.

Fig.1

Fig.2

| 1 | ADS | E1 | E2 | E3 | S | B |
|---|-----|----|----|----|---|---|

| 2 | B | ADS | E1 | E2 | E3 | S |
|---|---|-----|----|----|----|---|

| 3 | E3 | S | B | ADS | E1 | E2 |
|---|----|---|---|-----|----|----|

| 4 | E1 | E2 | E3 | S | B | ADS |
|---|----|----|----|---|---|-----|